# EUROPEAN PATENT APPLICATION

(11) **EP 3 048 546 A1**
(43) Date of publication of application: **27.07.2016**
(21) Application number: 15189282.5
(22) Date of filing: 12.10.2015
(51) Int. Cl.: G06F 19/00, H04N 1/32

(54) **MEDICAL IMAGE PROCESSING APPARATUS**

(30) Priority: 26.01.2015 JP 2015011950
(71) Applicant: Oki Data Corporation, Tokyo 108-8551 (JP)
(72) Inventor: Hirata, Masaru, Tokyo, 108-8551 (JP); Rabel, Patrick, CP 30527 94633 Rungis (FR)
(74) Representative: Piotrowicz, Pawel Jan Andrzej

(57) **Abstract**

An image processing apparatus includes a receiving unit that receives medical image data, a converter that converts the medical image data into a predetermined format, and an output unit having an association table storing an input source and an output destination in association with each other. The output unit outputs the medical image data received by the receiving unit to the output destination based on the association table.

## Description

### BACKGROUND OF THE INVENTION

The present invention relates to technology for printing an image to a medium based on medical image data in accordance with DICOM (Digital Imaging and Communications in Medicine) standard.

Generally, medical image data (DICOM data) created by a modality is accumulated in a server, and is printed as necessary. Upon printing, for example, the modality transmits a print command including identifiers specifying a server and data to the printer. Based on information of the identifiers, the printer acquires the medical image data from the server, and prints the medical image data on a medium (see, for example, Japanese Application Publication NO. 2001-94744).

Recently, with diversification of utilization forms of medical image data, it is desired to provide a flexible system construction.

### SUMMARY OF THE INVENTION

An aspect of the present invention is intended to provide a flexible system construction.

According to an aspect of the present invention, there is provided an image processing apparatus including a receiving unit that receives medical image data, a converter that converts the medical image data into a predetermined format, and an output unit having an association table storing an input source and an output destination of the medical image data in association with each other. The output unit outputs the medical image data received by the receiving unit to the output destination based on the association table.

Since the medical image data is outputted based on the association table storing the input source and the output destination of the medical image data in association with each other, a flexible system construction is provided.

### BRIEF DESCRIPTION OF THE DRAWINGS

In the attached drawings:
FIG. 1 is a block diagram showing a configuration of an image forming system including a DICOM printer according to the first embodiment of the present invention;
FIG. 2 is a block diagram showing a configuration of the DICOM printer according to the first embodiment;
FIG. 3 is a schematic view showing a basic configuration of DICOM data;
FIG. 4 is a view showing a transfer destination table according to the first embodiment;
FIG. 5 is a view showing an AET registration screen according to the first embodiment;
FIG. 6 is a view showing an AET setting screen according to the first embodiment;
FIG. 7 is a flowchart for illustrating an operation of the DICOM printer according to the first embodiment;
FIG. 8 is a block diagram showing an image forming system according to a modification of the first embodiment;
FIG. 9 is a view showing a transfer destination table according to the second embodiment of the present invention;
FIG. 10 is a view showing an AET setting screen according to the second embodiment;
FIG. 11 is a view showing a transfer destination table according to the third embodiment of the present invention;
FIG. 12 is a view showing an AET setting screen according to the third embodiment;
FIG. 13 is a block diagram showing an image forming system according to the fourth embodiment of the present invention;
FIG. 14 is a view showing a transfer destination table according to the fourth embodiment; and
FIG. 15 is a view showing an AET setting screen according to the fourth embodiment.

### DETAILED DESCRIPTION OF THE PREFERRED EMBODIMENT

### FIRST EMBODIMENT

### <CONFIGURATION OF IMAGE FORMING SYSTEM>

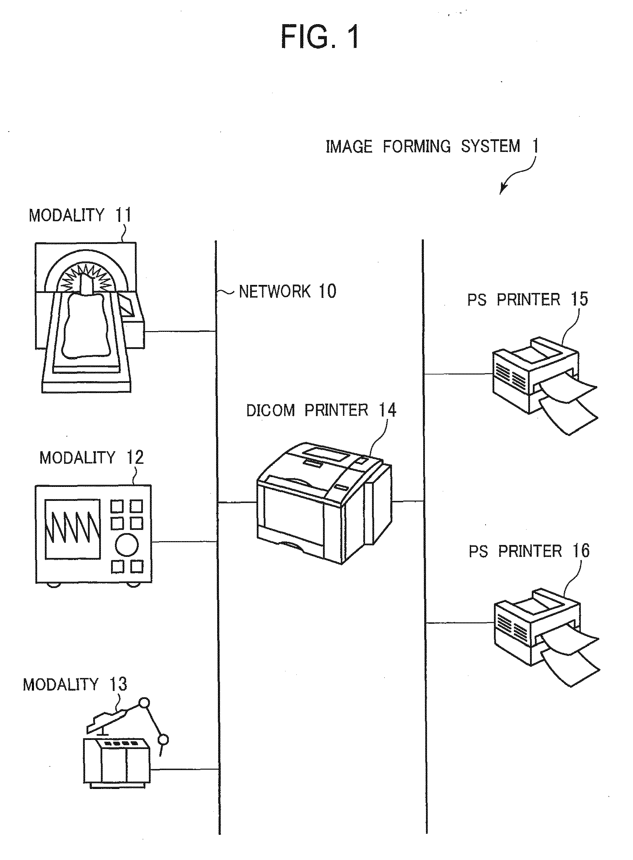

FIG. 1 is a block diagram showing a configuration of an image forming system as an image processing system, including an image forming apparatus as an image processing apparatus according to the first embodiment of the present invention. The image forming system 1 shown in FIG. 1 includes modalities 11, 12 and 13 as information processing apparatuses, a DICOM (Digital Imaging and Communications for Medicine) printer 14 as an image forming apparatus (i.e., an image processing apparatus, or a server), and a postscript printers 15 and 16 as image processing devices, and a network 10 such as a LAN (Local Area Network) or the like. The modalities 11, 12 and 13, the DICOM printer 14 and the postscript printers 15 and 16 are connected with each other through the network 10.

Each of the modalities 11, 12, and 13 photographs an image, and creates DICOM data (i.e., medical image data) in accordance with DICOM standard, and transmits the DICOM data to a specified apparatus through the network 10 in accordance with DICOM standard (i.e., DICOM communication protocol).

The modality 11 is, for example, a CT (Computed Tomography) apparatus. The modality 12 is, for example, an MR (Magnetic Resonance) apparatus. The modality 13 is, for example, a CR (Computed Radiography) apparatus. Each of the modalities 11, 12 and 13 has an operation panel operated by a user (for example, a doctor), and sends a service request (i.e., a demand for printing) to the DICOM printer 14 by user's operation.

Each of the postscript printers (hereinafter, referred to as PS printers) 15 and 16 is configured to receive postscript (trademark) data, expand the postscript data into bitmap data, and print the bitmap data on a medium such as a paper. The PS printers 15 and 16 may be of any kind as long as the PS printers 15 and 16 can perform printing based on postscript data. In this example, the PS printer uses electrophotography.

The DICOM printer 14 has a function to receive DICOM data transmitted from the modalities 11, 12 and 13 in accordance with the DICOM standard (i.e., DICOM communications protocol), and convert the DICOM data into postscript data, and performs printing on the medium or transfer the postscript data to the PS printer 15 or the PS printer 16.

That is, the DICOM printer 14 functions as a SPC (Service Class Provider) for SCUs (Service Class Users) such as the modalities 11, 12 and 13 and the like. The DICOM printer 14 as the SPC provides a service (i.e., printing of a medical image) in response to a service request from the SCU.

### <CONFIGURATION OF DICOM PRINTER>

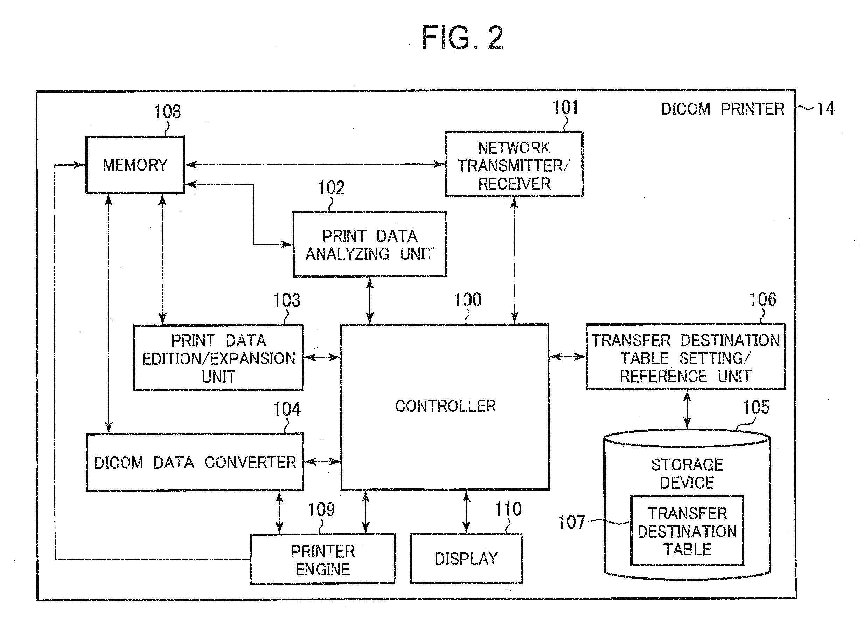

FIG. 2 is a block diagram showing a configuration of the DICOM printer 14. The DICOM printer 14 includes a controller 100, a network transmitter/receiver 101 as a receiving unit, a print data/command analyzer 102, and a print data edition/expansion unit 103, a DICOM data converter 104 as a converter, a storage device 105, a transfer destination table setting/reference unit 106 as a setting unit (or a reference unit), a memory 108, a printer engine 109 as an image forming unit, and a display 110.

Among these elements, the controller 100, the network transmitter/receiver 101, the print data/command analyzer 102, the print data edition/expansion unit 103, the DICOM data converter 104, and the transfer destination table setting/reference unit 106 are constituted by, for example, a CPU (Central Processing Unit). The memory 108 is constituted by, for example, a RAM (Random Access Memory).

The network transmitter/receiver 101 is configured to perform communication with the modalities 11, 12 and 13 through the network 10 using, for example, TCP/IP (Transmission Control Protocol/Internet Protocol). The network transmitter/receiver 101 receives the DICOM data from the modalities 11, 12 and 13 in accordance with the DICOM standard, and stores the received DICOM data in the memory 108.

The print data/command analyzer 102 is configured to analyze data stored in the memory 108 and stores the analysis result in the memory 108, according to an instruction of the controller 100.

The DICOM data converter 104 is configured to convert the DICOM data (stored in the memory 108 by the network transmitter/receiver 101) into the postscript data and stores the postscript data in the memory 108, according to an instruction of the controller 100.

The print data edition/expansion unit 103 is configured to translate the postscript data based on the analysis result stored in the memory 108, and edits and expands print data on the memory 108, according to an instruction of the controller 100.

The storage device 105 is constituted by, for example, a hard disk device. The storage device 105 stores the transfer destination table 107 described later.

The transfer destination table setting/reference unit 106 is configured to refer to the transfer destination table 107 stored in the storage device 105, and determine a transfer destination of the postscript data (i.e., a destination where the postscript data is to be transferred). The transfer destination table setting/reference unit 106 is also configured to accept user's input, and perform setting of the transfer destination table 107.

The printer engine 109 is configured to print the print data expanded on the memory 108 to the medium such as the paper. The printer engine 109 may use any kind of printing method. For example, the printer engine 109 is preferably configured to form a toner image (i.e., a developer image) on the medium using electrophotography.

In the case where the printer engine 109 uses electrophotography, the printer engine 109 includes an image bearing body such as a photosensitive drum, a charging portion that uniformly charges a surface of the image bearing body, an exposure device that emits light to expose the surface of the image bearing body so as to form a latent image, a developing device that develops the latent image to form a developer image, a transfer portion that transfers the developer image to the medium, and a fixing device that fixes the developer image to the medium. The exposure device may preferably be constituted by an LED (Light Emitting Diode) head which is suitable for forming high resolution image.

The display 110 is operated by a user to perform setting of the transfer destination table 107 via the transfer destination table setting/reference unit 106. The display 110 includes, for example, a touch panel or the like.

The controller 100 controls an entire operation of the DICOM printer 14. To be more specific, the controller 100 causes the DICOM data converter 104 to convert the DICOM data (received by the network transmitter/receiver 101) into the postscript data (i.e., page description language) that is printable by the PS printers 15 and 16. Further, the controller 100 determines a transfer destination (i.e., an output destination) based on the transfer destination table 107, and transfers the postscript data to the transfer destination (i.e., the PS printer 15, PS printer 16 or the like). The controller 100, the storage device 105 and the transfer destination table setting/reference unit 106 constitute an output unit.

### <CONFIGURATION OF DICOM DATA>

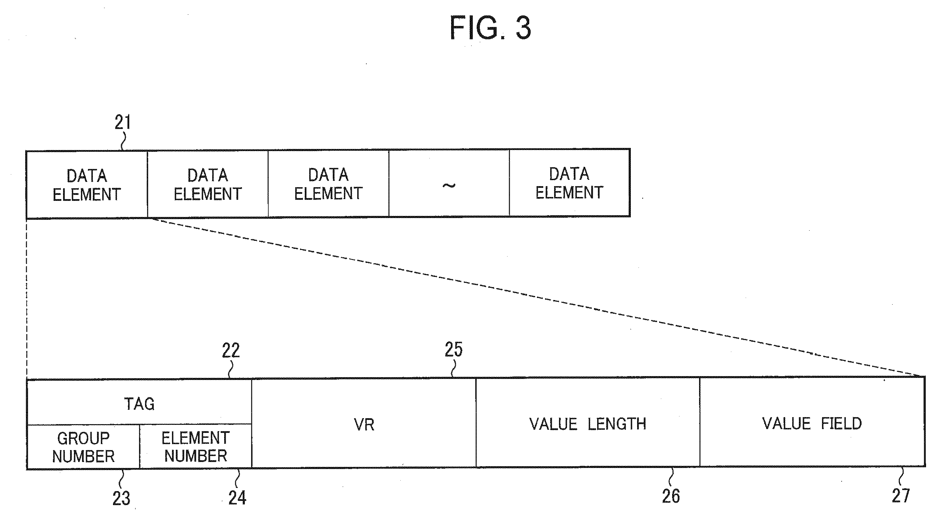

FIG. 3 is a schematic view showing a basic configuration of DICOM data. As shown in FIG. 3, the DICOM data includes a plurality of data elements 21. Each data element 21 includes a tag 22, a VR (Value Representation) 25, a Value Length 26 and a Value Field 27.

The tag 22 includes a group number 23 and an element number 24. A combination of the group number 23 and the element number 24 indicates a kind of information of data in the Value Field 27 of the data element 21. For example, when the group number 23 is "0010" and the element number 24 is "0010", the data in the Value Field 27 is a name of patient. When the group number 23 is "7fe0" and the element number 24 is "0010", the data in the Value Field 27 is a pixel data of a medical image.

The VR 25 indicates a data format (i.e., representation) of the data in the Value Field 27 of the data element 21. The VR 25 may be omitted in accordance with the standard. The Value Length 26 indicates a length (byte length) of the data in the Value Field 27.

The Value Field 27 is a data area. The data in the Value Field 27 may be pixel data of the medical image, or associated information. The associated information is, for example, a name, a birthday or an ID (Identification Data) of a patient, a photographing date, or a photographing region.

As described above, the DICOM data converter 104 (FIG. 2) of the DICOM printer 14 converts the DICOM data received from any of the modalities 11, 12 and 13 (FIG. 1) into the postscript data. Since the DICOM data includes the medical image and the associated information as described above, the DICOM data converter 104 creates the postscript data including the medical image and the associated information.

### <TRANSFER DESTINATION TABLE>

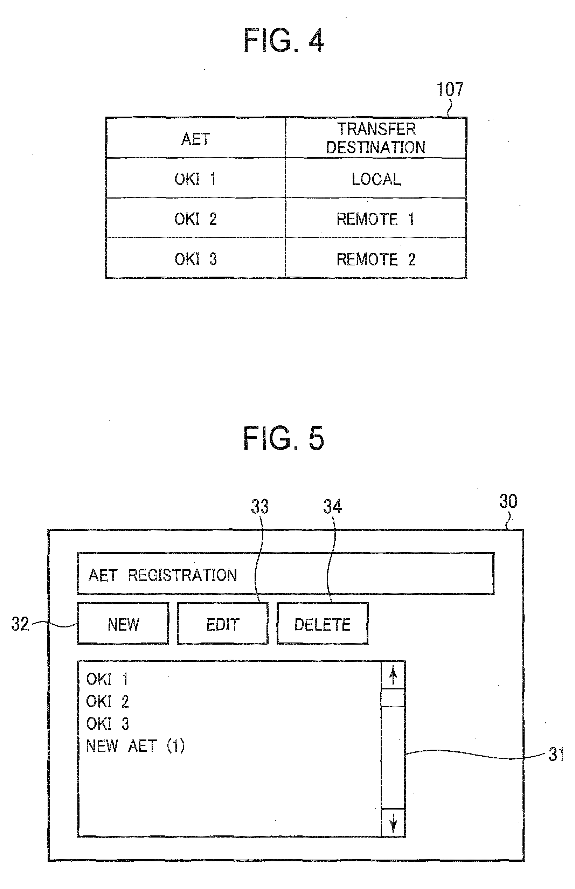

FIG. 4 is a view showing an example of the transfer destination table 107 (i.e., an association table) stored in the storage device 105. The transfer destination table 107 stores transfer destinations of the postscript data in association with AETs (Application Entity Titles).

Here, the AET named "OKI1" indicates printing of an image photographed by the modality 11. The AET named "OKI2" indicates printing of an image photographed by the modality 12. The AET named "OKI3" indicates printing of an image photographed by the modality 13.

In the transfer destination table 107, the AET "OKI1" is associated with the DICOM printer 14 (LOCAL) as a transfer destination (i.e., an output destination). The AET "OKI2" is associated with the PS printer 15 (Remote 1) as the transfer destination. The AET "OKI3" is associated with the PS printer 16 (Remote 2) as the transfer destination.

Each of the modalities 11, 12 and 13 transmits the DICOM Data while specifying the AET. The controller 100 (FIG. 2) of the DICOM printer 14 causes the transfer destination table setting/reference unit 106 to search the transfer destination table 107 based on the specified AET, determines the transfer destination (i.e., the output destination), and transmits the postscript data to the transfer destination (i.e., the PS printer 15, the PS printer 16 or the like).

In this regard, the AET corresponds to information indicating an input source of the DICOM data. In other words, the AET corresponds to information indicating a reception port (i.e., a port) at which the DICOM printer 14 receives the DICOM data. The AET also corresponds to information indicating a transmission source (i.e., the modalities 11, 12 and 13) of the DICOM data.

### <SETTING OF TRANSFER DESTINATION TABLE>

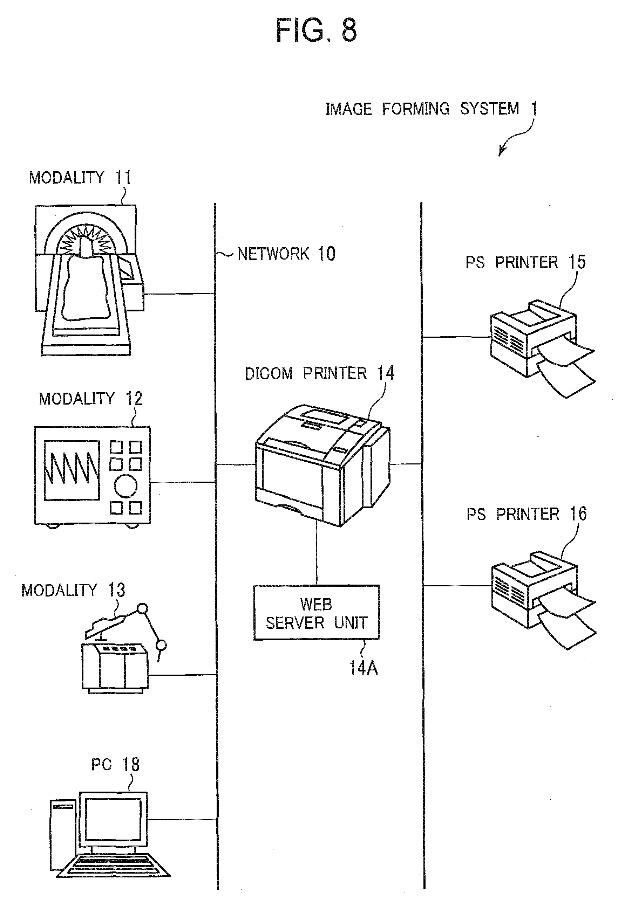

FIG. 5 is a view showing an AET registration screen 30 according to the first embodiment. The AET registration screen 30 is displayed by the transfer destination table setting/reference unit 106 to receive user's input. The AET registration screen 30 is displayed on, for example, the display 110 of the DICOM printer 14. However, this embodiment is not limited to such a configuration. For example, it is also possible to employ a configuration that receives user's input via a personal computer or the like using a Web service. This will be described later with reference to FIG. 8.

The AET registration screen 30 includes an AET list 31, a new button 32, an edit button 33 and a delete button 34.

The AET list 31 includes a list of AETs which are currently registered. Here, the AETs "OKI1", "OKI2" and "OKI3" are registered in the AET list 31.

The new button 32 is a button pressed to add an AET with a unique name to the AET list 31. In an example shown in FIG. 5, the AET with the unique name "NewAET (1)" is added to the AET list 31.

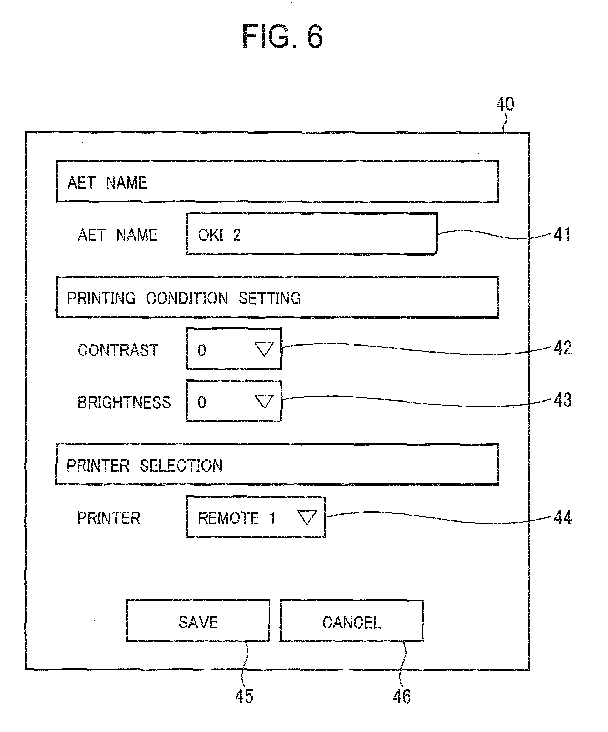

The edit button 33 is a button pressed to change a setting of the AET selected in the AET list 31. When the edit button 33 is pressed, the AET registration screen 30 shifts to an AET setting screen 40 (FIG. 6) described below. The delete button 34 is a button pressed to delete the AET selected in the AET list 31.

FIG. 6 is a view showing the AET setting screen 40 according to the first embodiment. The AET setting screen 40 is used to perform setting of printing conditions for the AET. The AET setting screen 40 is displayed on the display 110 of the DICOM printer 14 when the user presses the edit button 33 of the AET registration screen 30 (FIG. 5).

The AET setting screen 40 includes an AET name section 41, a contrast setting section 42, a brightness setting section 43, a printer setting section 44, a save button 45 and a cancel button 46.

The AET name section 41 displays the AET name (i.e., a setting object) selected in the AET list 31 of the AET registration screen 30. The AET name section 41 is configured to allow text input so that the AET name (displayed in the AET name section 41) can be changed as necessary.

The contrast setting section 42 is configured so that a contrast (i.e., a difference in brightness) of a print image can be set, for example, on a scale from -5 to +5. The brightness setting section 43 is configured so that a brightness of the print image can be set, for example, on a scale from -5 to +5. In this regard, it is also possible to set other printing conditions than the contrast and the brightness.

The printer setting section 44 is a setting section for specifying a printer that prints the DICOM data or the postscript data. The printer setting section 44 includes, for example, a scroll bar. The printer setting section 44 allows the user to select any of the DICOM printer 14, the PS printer 15 and the PS printer 16. In the example shown in FIG. 4, the PS printer 15 (Remote 1) is selected for the AET "OKI2".

The save button 45 is a button pressed to confirm settings that have been set on the AET setting screen 40, and store the settings in the storage device 105. When the save button 45 is pressed, the setting in the printer setting section 44 is stored in the transfer destination table 107 of the storage device 105 by the transfer destination table setting/reference unit 106. Further, the settings in the contrast setting section 42 and the brightness setting section 43 are stored in the storage device 105. After the settings are stored in the storage device 105, the AET setting screen 40 (FIG. 6) shifts to the AET registration screen 30 (FIG. 5).

The cancel button 46 is a button pressed to cancel the settings that have been set on the AET setting screen 40. When the cancel button 46 is pressed, the setting in the AET setting screen 40 is cancelled, and the AET setting screen 40 (FIG. 6) shifts to the AET registration screen 30 (FIG. 5).

### <OPERATION OF DICOM PRINTER>

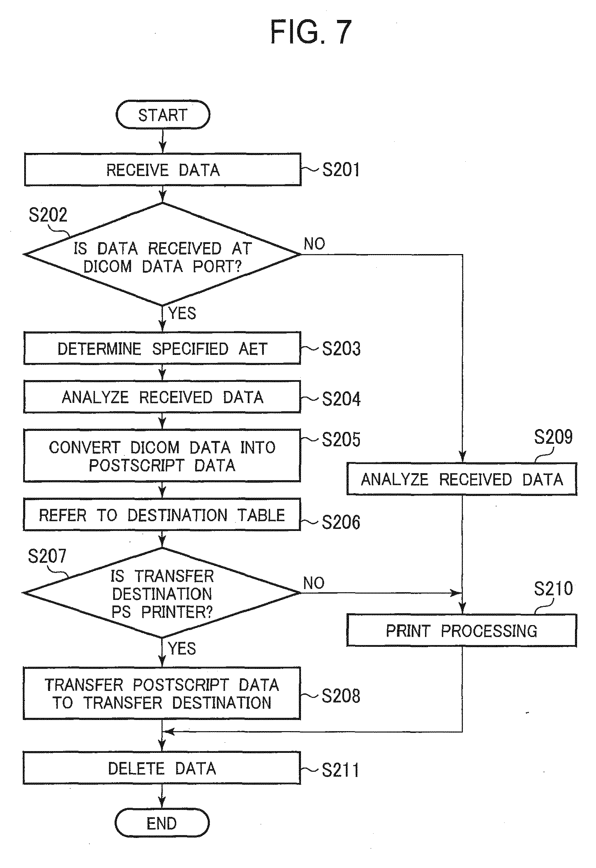

Next, an operation of the DICOM printer 14 according to the first embodiment will be described. FIG. 7 is a flowchart showing the operation of the DICOM printer 14.

Description will be made of a case where data is transmitted from the modality 11, the modality 12, the modality 13 or a personal computer to the DICOM printer 14 through the network 10.

In this regard, when the modality transmits the DICOM data, the modality specifies an IP address and a port number. When the personal computer transmits normal data (for example, postscript data), the personal computer specifies an IP address and a port number.

There are two types of port numbers: a port number for DICOM data, and a port number for normal data. When the modality transmits the DICOM data, the modality specifies the port number for DICOM data, and also specifies the AET.

First, the network transmitter/receiver 101 of the DICOM printer 14 receives data transmitted from the modality or the personal computer through the network 10 (step S201). If the received data is the DICOM data, association establishment is performed, but description of the association establishment is herein omitted.

Then, the controller 100 determines whether the data received by the network transmitter/receiver 101 is received at the port number for DICOM data (step S202).

If the data is received at the port number for DICOM data (YES in step S202), the controller 100 determines which of the AETs is specified (step S203). As described above, the DICOM data is transmitted by the modality along with specifying of the AET.

Then, the controller 100 causes the print data/command analyzer 102 to analyze the DICOM data, and store the analysis result in the memory 108 (step S204).

Then, the controller 100 creates the postscript data as a page description language based on the DICOM data (stored in the memory 108 by the network transmitter/receiver 101) (step S205). That is, the controller 100 causes the DICOM data converter 104 to convert the DICOM data into the postscript data.

In this regard, the postscript data may be created by converting the DICOM data based on the printing conditions (for example, contrast and brightness) set on the AET setting screen 40 (FIG. 6). In this case, the printing conditions may be taking into account by, for example, the DICOM printer 14. The postscript data may also be created so as to include a converting instruction/command corresponding to the printing conditions set on the AET setting screen 40 and the DICOM data. In this case, the printing conditions may be taking into account by, for example, the PS printer 15 or the PS printer 16.

Then, the controller 100 causes the transfer destination table setting/reference unit 106 to refer to the transfer destination table 107 (FIG. 2) stored in the storage device 105, and search a printer to which the postscript data is to be transferred (step S206).

To be more specific, the controller 100 notifies the transfer destination table setting/reference unit 106 of the AET determined in the above described step S203. The transfer destination table setting/reference unit 106 searches the transfer destination table 107 based on the notified AET, and selects the transfer destination corresponding to the notified AET.

For example, when the AET is "OKI1", the DICOM printer 14 (LOCAL) is selected based on the transfer destination table 107. When the AET is "OKI2", the PS printer 15 (Remote 1) is selected. When the AET is "OKI3", the PS printer 16 (Remote 2) is selected.

Then, based on a search result by the transfer destination table setting/reference unit 106, the controller 100 checks whether the transfer destination selected in step S206 is either the PS printer 15 and the PS printer 16 or the DICOM printer 14 (step S207).

If the transfer destination is the DICOM printer 14 (NO in step S207), the controller 100 causes the DICOM printer 14 to perform print processing. That is, the controller 100 causes the print data edition/expansion unit 103 to translate the postscript data based on the analysis result stored in the memory 108 into print data. The print data edition/expansion unit 103 edits and expands the print data, and stores the print data in the memory 108. The printer engine 109 prints the expanded print data on the medium such as the paper (step S210).

If the transfer destination is the PS printer 15 or the PS printer 16 (YES in Step S207), the controller 100 transfers the postscript data (converted in step S205) to the printer as the transfer destination (i.e., the PS printer 15 or PS printer 16) through the network transmitter/receiver 101 (step S208).

The printer as the transfer destination (i.e., the PS printer 15 or the PS printer 16) prints the print data transmitted from the DICOM printer 14 on the medium such as the paper.

After the transfer of the print data is completed, the controller 100 deletes the print data stored in the memory 108 (step S211).

In contrast, in step S202, if the received data is received at the normal data port number (NO in step S202), the controller 100 causes the print data/command analyzer 102 to analyze the received data (step S209). Then, the controller 100 proceeds to the above described step S210, and causes the printer engine 109 to perform print processing.

### <ADVANTAGES OF FIRST EMBODIMENET>

According to the first embodiment of the present invention, the DICOM printer 14 includes the transfer destination table 107 storing the input source and the output source of the DICOM data in association with each other. The DICOM printer 14 convers the received DICOM data into the postscript data, and transfers the postscript data to the transfer destination (i.e., the PS printer 15, the PS printer 16 or the like) based on the transfer destination table 107. Therefore, a flexible system construction can be provided.

That is, print processing conventionally performed by a single DICOM printer can be shared by a plurality of printers. Therefore, when a certain printer is incapable of printing due to toner exhaustion or it takes time until the printer starts printing, another printer can be used to perform printing.

Further, for example, a medical image photographed by each modality can be printed using the PS printer disposed near the modality (for example, disposed in the same consultation room as the modality). Therefore, convenience is enhanced.

Further, the DICOM printer 14 converts the DICOM data into the postscript data, and transfers the postscript data to the PS printer as the transfer destination. Therefore, medical images can be printed using the PS printers 15 and 16 which are relatively inexpensive. That is, a cost reduction can be achieved.

Further, the transfer destination table 107 stores the AETs and the transfer destinations (i.e., the PS printer 15, the PS printer 16 and the like). The AETs corresponds to input sources of the DICOM data. Therefore, it becomes possible to select the transfer destination associated with the input source of the DICOM data.

Further, the transfer destination table setting/reference unit 106 is configured to perform setting of the transfer destination table 107 based on user's operation. Therefore, it becomes possible to set the transfer destination table 107 according to utilization by the user. That is, convenience is enhanced.

### MODIFICATION.

In the above described first embodiment, the setting of the transfer destination table 107 is performed on the display 110 of the DICOM printer 14. However, the present invention is not limited to such a configuration. For example, as shown in FIG. 8, the DICOM printer 14 may be provided with a WEB server unit 14A. The WEB server unit 14A may provide a Web Service through the network 10 using an HTTP (Hypertext Transfer Protocol). The setting of the transfer destination table 107 may be performed using a personal computer 18 (i.e., an input unit) connected to the network 10. In this case, the AET registration screen 30 (FIG. 5) and the AET setting screen 40 (FIG. 6) may be displayed on a display of the personal computer 18.

Further, in the above described embodiment, a single AET is associated with a single printer. However, the present invention is not limited to such a configuration. For example, a plurality of AETs may be associated with a single printer. For example, the modalities 11 and 12 can share the PS printer 15. With such a configuration, in a medical examination, diagnostic results photographed by a plurality of modalities can be outputted by a single printer, and can be delivered to a patient.

### SECOND EMBODIMENT.

Next, the second embodiment of the present invention will be described. In the above described first embodiment, a single AET is associated with a single printer. However, the second embodiment relates to a configuration in which a single AET can be associated with a plurality of printers.

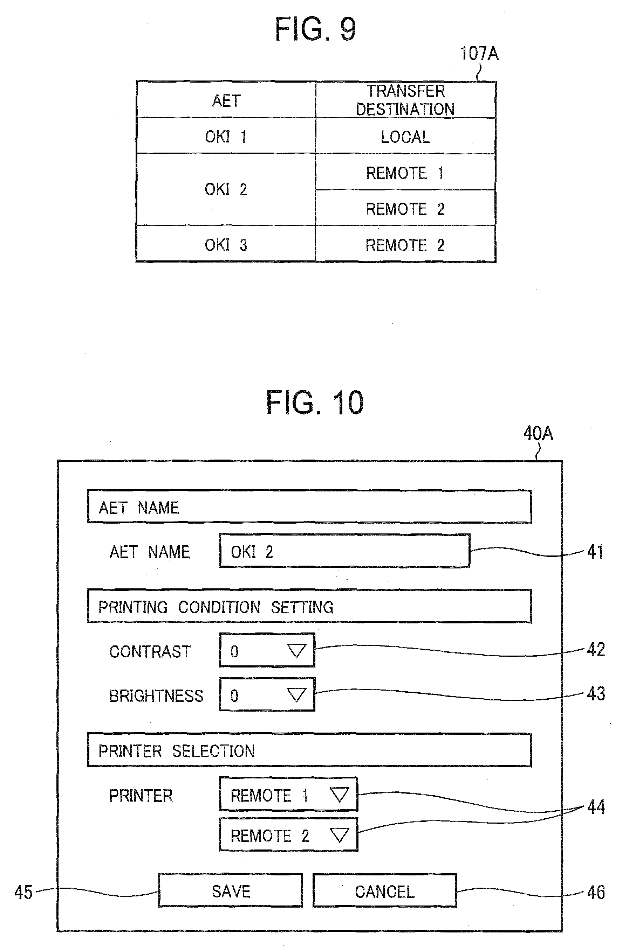

FIG. 9 is a view showing an example of a transfer destination table 107A stored in the storage device 105 according to the second embodiment. In the transfer destination table 107A, the AET "OKI1" is associated with the DICOM printer 14 (LOCAL) as the transfer destination (i.e., an output destination). The AET "OKI2" is associated with the PS printer 15 (Remote 1) and the PS printer 16 (Remote 2) as the destinations. The AET "OKI3" is associated with the PS printer 16 (Remote 2) as the transfer destination.

FIG. 10 is a view showing an AET setting screen 40A according to the second embodiment. The AET setting screen 40A includes the AET name section 41, the contrast setting section 42, the brightness setting section 43, the printer setting section 44, the save button 45 and the cancel button 46, as with the AET setting screen 40 (FIG. 6) described in the first embodiment.

In the AET setting screen 40A, the printer setting section 44 is so configured that a plurality of printers (in this example, two printers) can be selected. Here, for example, the PS printer 15 (Remote 1) and the PS printer 16 (Remote 2) are selected for the AET "OKI2". Other configurations of the AET setting screen 40A are the same as those of the AET setting screen 40 (FIG. 6) described in the first embodiment. The settings that have been set on the printer setting section 44 are stored in the transfer destination table 107A of the storage device 105 by the transfer destination table setting/reference unit 106.

Here, a single AET can be associated to two printers. However, it is also possible to employ a configuration in which a single AET can be associated with three or more printers.

According to the second embodiment, a plurality of printers can be selected for a single AET, and therefore various operations can be performed. For example, a medical image photographed by a single modality can be printed by a plurality of printers. Therefore, it becomes possible to flexibly respond to utilization by the user.

Further, it is also possible to employ a configuration that prompts the user to select one of the printers (associated with the AET) for printing. For example, if there are a plurality of places for a doctor (i.e., an outpatient clinic or an inpatient's ward) and if the plurality of places are provided with respective printers, the doctor can select one printer according to his/her schedule.

### THIRD EMBODIMENT

Next, the third embodiment of the present invention will be described. The third embodiment relates to a configuration that allows selection of a tray (i.e., a medium feeding portion) of the printer for the AET.

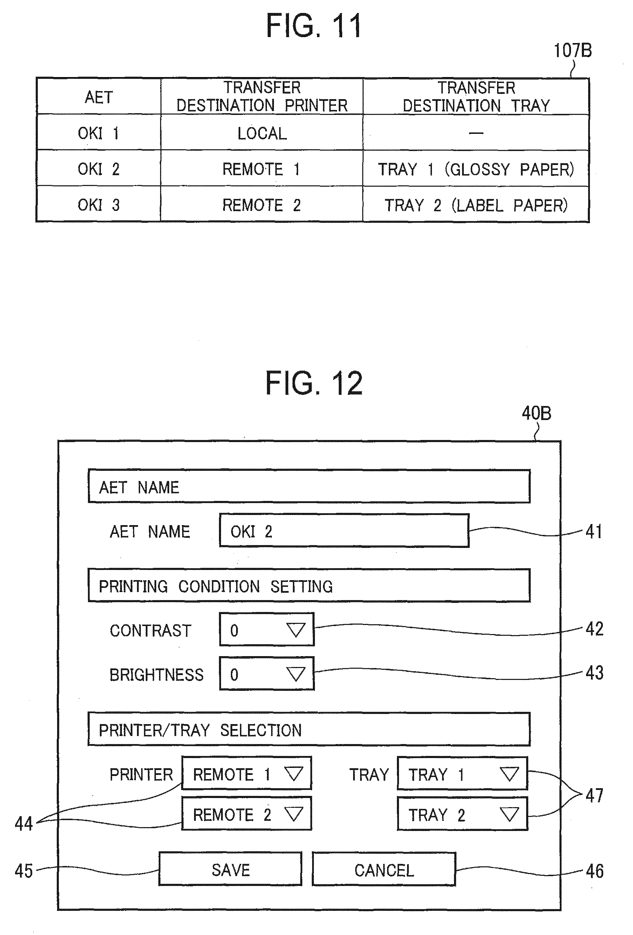

FIG. 11 is a view showing an example of a transfer destination table 107B stored in the storage device 105 according to the third embodiment. In the transfer destination table 107B, the AET "OKI1" is associated with the DICOM printer 14 (LOCAL) as the transfer destination (i.e., the output destination). The AET "OKI2" is associated with a tray 1 (storing glossy papers) of the PS printer 15 (Remote 1) as the transfer destination. The AET "OKI3" is associated with the tray 2 (storing label papers) of the PS printer 16 (Remote 2) as the transfer destination.

FIG. 12 is a view showing an AET setting screen 40B according to the third embodiment. The AET setting screen 40B includes the AET name section 41, the contrast setting section 42, the brightness setting section 43, the printer setting section 44, the save button 45 and the cancel button 46, as with the AET setting screen 40 (FIG. 6) described in the first embodiment.

The AET setting screen 40B includes a tray setting section 47 in addition to the printer setting section 44. The tray setting section 47 includes, for example, a scroll bar. The AET setting screen 40B is configured so that one of the tray 1 and the tray 2 of the PS printer 15 (Remote 1) can be selected, and one of the tray 1 and the tray 2 of the PS printer 16 (Remote 2) can be selected. Other configurations of the AET setting screen 40B are the same as those of the AET setting screen 40 (FIG. 6) described in the first embodiment. The settings that have been set on the printer setting section 44 and the tray setting section 47 are stored in the transfer destination table 107B of the storage device 105 by the transfer destination table setting/reference unit 106.

In this example, the AET setting screen 40B is so configured that one of two trays of each of the PS printer 15 and the PS printer 16 can be selected. However, it is also possible to employ a configuration that allows selection of one of three or more trays.

According to the third embodiment, the tray of the printer can be selected for the AET. Therefore, it becomes possible to select the tray storing optimal media (i.e., glossy papers, plain papers, label papers, or the like) in consideration of, for example, a precision of a medical image or the like. Therefore, it becomes possible to flexibly respond to utilization by the user.

### FOURTH EMBODIMENT.

Next, the fourth embodiment of the present invention will be described. The fourth embodiment relates to a configuration in which a USB (Universal Serial Bus) memory 19 as a storage medium can be selected as the transfer destination (i.e., the output destination) of the DICOM data, as well as the PS printers 15 and 16.

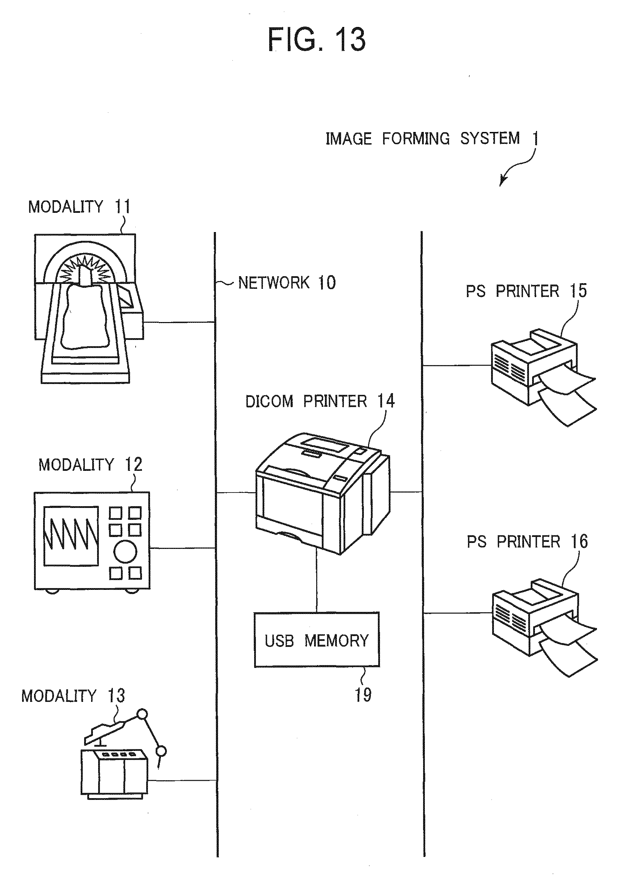

FIG. 13 is a view showing an image forming system (i.e., an image processing system) according to the fourth embodiment. The image forming system of the fourth embodiment includes a USB memory 19 as a storage medium in addition to elements of the image forming system (FIG. 1) described in the first embodiment. The USB memory 19 is mounted to the DICOM printer 14. In this regard, this embodiment is not limited to a configuration having the USB memory 19. For example, the USB memory 19 may be replaced with a storage device connected to the DICOM printer 14 through the network 10.

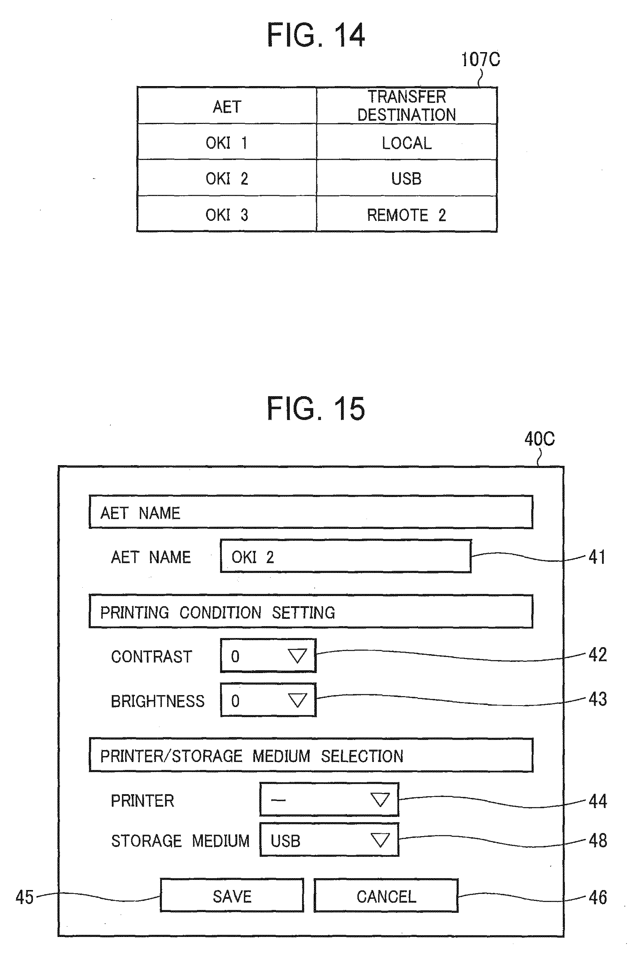

FIG. 14 is a view showing an example of a transfer destination table 107C stored in the storage device 105 according to the fourth embodiment. In the transfer destination table 107B, the AET "OKI1" is associated with the USB memory 19 as the transfer destination (i.e., the output destination). The AET "OKI2" is associated with the PS printer 15 (Remote 1) as the transfer destination. AET "OKI3" is associated with the PS printer 16 (Remote 2) as the transfer destination.

FIG. 15 is a view showing an AET setting screen 40C according to the fourth embodiment. The AET setting screen 40C includes the AET name section 41, the contrast setting section 42, the brightness setting section 43, the printer setting section 44, the save button 45 and the cancel button 46, as with the AET setting screen 40 (FIG. 6) described in the first embodiment.

The AET setting screen 40C includes, a storage medium setting section 48 in addition to the printer setting section 44. The storage medium setting section 48 includes, for example, a scroll bar. The storage medium setting section 48 is so configured that the USB memory 19 (as well as the PS printers 15 and 16) can be selected. The storage medium setting section 48 may also be configured so that one of a plurality of storage media can be selected. Other configurations of the AET setting screen 40C are the same as those of the AET setting screen 40 (FIG. 6) described in the first embodiment. The settings that have been set on the printer setting section 44 and the storage medium setting section 48 are stored in the transfer destination table 107C of the storage device 105 by the transfer destination table setting/reference unit 106.

According to the fourth embodiment, the postscript data converted from the DICOM data can be outputted to the storage medium such as the USB memory 19. Therefore, it becomes possible to respond to various types of utilization. For example, the postscript data stored in the USB memory 19 or the like can be printed by another printer.

In the above described fourth embodiment, the USB memory 19 is described as an example of the storage medium. However, it is also possible to employ a configuration in which a data management device (for example, a file server) can be selected as the transfer destination. With such a configuration, for example, the postscript data can be printed and stored in the data management device. Further, in this case, it is also possible to employ a configuration that does not only store the postscript data (converted from the DICOM data) but also stores the DICOM data itself in the data management device. Furthermore, it is also possible to employ a configuration that prompts the user to select either one or both of printing and storing the data (i.e., the postscript data or the DICOM data).

The above described embodiments and modifications may be combined suitably.

In the above described embodiments and modifications, the DICOM printer 14 determines the transfer destination (i.e., the output destination) of the DICOM data. However, the present invention is not limited to such a configuration. For example, a server such as a personal computer can also be used instead of the DICOM printer 14. In this case, the server may convert DICOM data into postscript data, and output the postscript data to the PS printer 15 and/or the PS printer 16 (or USB memory 19) based on the transfer destination table 107.

Further, an image forming apparatus such as an MFP (Multi-Function Peripheral) may be provided with functions of the DICOM printer 14 of the above described embodiments and modifications.

Further, although the LAN is used as the network in the above described embodiments and modifications, the network 10 is not limited to the LAN. Furthermore, the kinds and the number of the modalities are not limited to those described above.

While the preferred embodiments of the present invention have been illustrated in detail, it should be apparent that modifications and improvements may be made to the invention without departing from the spirit and scope of the invention as described in the following claims.

## Claims

1. An image processing apparatus (14) comprising:
a receiving unit (101) that receives medical image data;
a converter (104) that converts the medical image data into a predetermined format; and
an output unit (100, 105, 106) having an association table (107) storing an input source (11, 12, 13) and an output destination (15, 16) in association with each other,
wherein the output unit (100, 105, 106) outputs the medical image data received by the receiving unit (101) to the output destination (15, 16) based on the association table (107).

2. The image processing apparatus (14) according to claim 1, wherein the receiving unit (101) receives the medical image data in accordance with DICOM standard; and
wherein the converter (104) converts the medical image data received by the receiving unit (101) into postscript data.

3. The image processing apparatus (14) according to claim 1 or 2, wherein the association table (107) is changeable by user's operation.

4. The image processing apparatus (14) according to claim 2, wherein AETs are specified for respective sets of the medical image data received by the receiving unit (101); and
wherein the association table (107) stores the AETs and the output destinations (15, 16) in association with each other.

5. The image processing apparatus (14) according to claim 4, wherein the association table (107) stores each of the AETs in association with one of the output destinations (15, 16).

6. The image processing apparatus (14) according to claim 4, wherein the association table (107) stores at least one of the AETs in association with a plurality of the output destinations (15, 16).

7. The image processing apparatus (14) according to any one of claims 4 to 6, wherein at least one of the output destinations (15, 16) includes a medium feeding portion that feeds a medium on which the medical image data is to be printed,
wherein the association table (107) stores at least one of the AETs in association with the medium feeding portion.

8. The image processing apparatus (14) according to any one of claims 4 to 7, wherein the association table (107) stores at least one of the AETs in association with a recording medium.

9. The image processing apparatus (14) according to any one of claims 1 to 8, wherein the association table (107) stores the image processing apparatus (14) as the output destinations.

10. The image processing apparatus (14) according to any one of claims 1 to 9, further comprising a storage device (105) that stores the association table (107).
